# FASCICULE DE BREVET EUROPEEN

(11) **EP 1 674 069 B1**
(45) Date de publication et mention de la délivrance du brevet: **28.05.2008**
(21) Numéro de dépôt: 05292390.1
(22) Date de dépôt: 10.11.2005
(51) Int. Cl.: A61K 8/26, A61Q 19/08

(54) **Procédé cosmétique de soin de la peau et kit associé**
Kosmetisches Verfahren zur Pflege der Haut und dazu geeigneter Kit
Cosmetic method for performing skin care and kit therefor

(30) Priorité: 17.12.2004 FR 0453043
(43) Date de publication de la demande: 28.06.2006
(73) Titulaire: L'ORÉAL, 75008 Paris (FR)
(72) Inventeur: Marion, Catherine, 92160 Antony (FR)
(74) Mandataire: Leonard, Armelle

(56) Documents cités:
- RU-C1- 2 203 040
- US-A- 5 516 517

## Description

La présente invention se rapporte à un procédé cosmétique de soin de la peau, destiné à atténuer les signes cutanés du vieillissement, comprenant l'application successive sur la peau préalablement mouillée de quatre compositions, à savoir : une composition de microdermabrasion, une composition de peeling, une composition apaisante et une composition anti-âge ayant une constitution donnée.

Elle se rapporte également à un kit comprenant quatre types de conditionnement dont chacun renferme l'une des compositions précitées.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules.

Jusqu'à présent, on traitait les rides et les ridules à l'aide de produits cosmétiques contenant des actifs agissant soit à la surface de la peau, soit au niveau dermique.

On a ainsi proposé d'utiliser des actifs ayant un effet de renouvellement des cellules épidermiques et/ou un effet kératolytique, véhiculés dans des compositions non rincées (généralement sous forme d'hydroxyacides), ou encore dans des compositions de peeling destinées à être mises en oeuvre dans le cadre privé ou chez un dermatologue (selon la profondeur du peeling). A côté de ces desquamants "chimiques", il est connu d'utiliser des abrasifs tels que l'oxyde d'aluminium pour exfolier la surface de la peau. Ce procédé d'exfoliation est connu sous le terme de "microdermabrasion". La microdermabrasion est notamment mise en oeuvre par des esthéticiennes ou des dermatologues qui ont recours à des micro-cristaux d'oxyde d'aluminium pour resurfacer les couches superficielles de la peau. Ces cristaux sont projetés sur la peau au moyen d'un appareil qui les aspire ensuite avec la peau abrasée.

Parmi les actifs ayant un effet dermique, il est connu d'apporter à la peau des actifs favorisant la synthèse, ou prévenant la dégradation, des fibres élastiques (collagène et élastine) ou des glycosaminoglycannes, qui composent le tissu cutané.

Il n'en demeure pas moins qu'il subsiste le besoin de disposer d'une méthode permettant de lutter efficacement contre les rides, qui ait des effets visibles rapidement et une action à long terme.

Le document brevet RU-C1-2 203 040 décrit un procédé cosmétique de soin de la peau, comprenant l'application successive sur une zone de peau:
- d'une composition comprenant une eau minérale,
- d'une composition renfermant de l'argile (un abrasif léger).

Le document brevet US-A-5 516 517 décrit un procédé cosmétique de soin de la peau, destiné à atténuer les signes cutanés du vieillissement, comprenant l'application successive sur une zone de peau:
- d'une composition de peeling enzymatique (a),
- d'une composition anti-âge renfermant un dérivé de retinol (c) et (d).

Or, la Demanderesse a découvert qu'il était possible de satisfaire ce besoin en suivant un protocole mettant en oeuvre quatre compositions appliquées successivement.

La présente invention a ainsi pour objet un procédé cosmétique de soin de la peau, destiné à atténuer les signes cutanés du vieillissement, comprenant l'application successive sur une zone de peau préalablement mouillée :
d'une composition de microdermabrasion renfermant au moins 5% en poids de particules d'oxyde métallique, qui est appliquée par massage, de préférence pendant au moins une minute, puis éliminée par lavage,
- d'une composition de peeling renfermant au moins 3% en poids d'au moins un agent desquamant,
- d'une composition apaisante comprenant une eau thermale ou minérale ayant une minéralisation d'au moins 300 mg/l,
- d'une composition anti-âge renfermant au moins un actif anti-âge choisi parmi : les composés augmentant la synthèse de collagène et/ou d'élastine et/ou des glycosaminoglycannes et/ou des protéoglycannes et/ou de la fibronectine et/ou de la laminine ; les composés inhibant la dégradation du collagène et/ou de l'élastine ; les agents dermo-décontractants ; les agents inhibant la glycation des protéines ; les agents augmentant la prolifération des kératinocytes et/ou des fibroblastes ; les agents augmentant la différenciation des kératinocytes ; et leurs mélanges.

De préférence, la zone de peau sur laquelle le procédé ci-dessus est mis en oeuvre est une zone de peau du visage, de préférence tout le visage excepté le contour des yeux.

La première étape du procédé selon l'invention, ci-après désignée par étape de microdermabrasion, est un type d'exfoliation destiné à resurfacer les couches superficielles de la peau. Cette étape consiste à appliquer sur la peau une composition renfermant au moins 5% en poids de particules d'oxyde métallique, qui peuvent par exemple être enrobées, notamment d'un composé siliconé, mais sont de préférence non enrobées.

Il s'agit avantageusement d'un oxyde de magnésium ou d'aluminium. Ces particules présentent avantageusement une pureté d'au moins 95%, mieux, d'au moins 99%. Leur taille moyenne de particule va de préférence de 100 à 180 µm. L'oxyde d'aluminium est préféré pour une utilisation dans la présente invention, en particulier sous forme anhydre cristallisée (corindon).

Comme particules d'oxyde d'aluminium, on peut utiliser des particules calcinées à haute température, jusqu'à obtenir la structure cristalline du corindon (α-Al₂O₃), puis traitées pour former des grains pourvus d'arêtes tranchantes et ayant une distribution de tailles de particules donnée, les particules ayant de préférence un diamètre de particule moyen compris entre 100 et 180 µm et de préférence entre 130 et 150 µm. Leur distribution est avantageusement telle qu'aucune des particules n'a un diamètre supérieur à 250 µm. De telles particules sont notamment disponibles dans le commerce auprès de la société MARKETECH INTERNATIONAL sous la dénomination commerciale Dermagrain. Les particules référencées Dermagrain 900 sont constituées d'alumine alpha cristallisée pure à 99,55%, présentant un diamètre moyen de particule d'environ 140 µm, les particules ayant toutes un diamètre inférieur à 250 µm. Moins de 3% des particules a un diamètre inférieur à 105 µm. D'autres particules sont disponibles auprès de la société INDUSTRIAL SUPPLY sous la dénomination commerciale ARL100 et ARL120. Il s'agit de particules d'oxyde d'aluminium ayant une taille moyenne de particule de 120 et 100 µm, respectivement, et une distribution de tailles de particules allant de 75 à 212 µm et de 63 à 180 µm, respectivement.

En variante, l'oxyde métallique utilisé dans la composition selon l'invention peut être de l'oxyde de magnésium ayant de préférence une taille moyenne de particule allant de 100 à 180 µm. Un exemple de particules de ce type est commercialisé par la société MarkeTech International sous la dénomination commerciale Magnaderm 100. Il s'agit de particules ayant un diamètre moyen de particule d'environ 120 µm et une pureté d'au moins 99%.

L'oxyde métallique peut représenter de 5 à 40%, et de préférence de 10 à 30%, et plus préférentiellement 20%, du poids total de la composition mise en oeuvre dans la première étape du procédé selon l'invention. Cette composition a de préférence un pH supérieur à 4 et inférieur à 8, mieux, inférieur ou égal à 7 et, encore mieux, compris entre 5,5 et 7.

La composition utilisée dans la première étape du procédé selon l'invention peut se présenter sous forme de lotion, de gel, de fluide ou de crème. Elle peut comprendre différents adjuvants et renferme avantageusement au moins un polyholoside hétérogène. La Demanderesse a en effet démontré que ces composés permettaient de réduire l'inconfort associé à l'utilisation des particules abrasives d'oxyde métallique précitées. Ce polyholoside hétérogène peut être un alginate, ou encore un polyholoside hétérogène comprenant au moins un motif fucose, comprenant notamment des motifs fucose, galactose et acide galacturonique, en particulier un enchaînement linéaire de α-L-Fucose, de α-D-Galactose et d'acide galacturonique. Un tel polyholoside est notamment disponible-sous forme d'une solution à 1% dans l'eau auprès de la société SOLABIA sous la dénomination commerciale Fucogel 1000 PP^{®}.

L'application de cette composition peut se faire par projection de la composition sur la peau au moyen d'un appareil qui l'aspire ensuite avec la peau abrasée. En variante, cette composition peut être appliquée sur la peau par massage manuel du bout des doigts, ou par massage mécanique au moyen d'un appareil vibrant pourvu d'une tête de massage munie d'un tampon, tel que décrit dans la demande US 2001/0046506 ou le brevet US-6,652,888, par exemple. La composition est ensuite éliminée par lavage. Pour ce faire, la peau sera généralement rincée à l'eau.

La seconde étape du procédé selon l'invention, ou étape de peeling, comprend l'application sur la peau d'une composition renfermant au moins 3% en poids d'au moins un agent desquamant.

Cet agent desquamant peut être notamment choisi parmi : les α-hydroxyacides tels que l'acide citrique, lactique, glycolique, malique, tartrique ou mandélique ; les β-hydroxyacides tels que l'acide salicylique ou l'acide n-octanoyl-5-salicylique ; l'urée ; les composés aminosulfoniques et en particulier l'acide (N-2 hydroxyéthylpiperazine-N-2-éthane) sulfonique (HEPES) ; et les dérivés de l'acide 2-oxothiazolidine-4-carboxylique (procystéine). L'acide lactique, l'acide glycolique, l'HEPES et leurs mélanges sont préférés pour une utilisation dans la présente invention.

La quantité d'agent desquamant peut représenter de 3 à 15% du poids de la composition mise en oeuvre dans la seconde étape du procédé selon l'invention. On peut ainsi utiliser 10% d'acide glycolique, ou un mélange comprenant 3,5% d'acide glycolique ; 0,5% d'acide lactique et 5% d'HEPES, par rapport au poids total de la composition.

Cette composition a de préférence un pH compris entre 2,5 et 6, avantageusement entre 3,5 et 4,5.

Elle peut être appliquée sur la peau sous forme de lotion, de gel, de fluide ou de crème. En variante, elle peut être appliquée sous forme de masque ou de patch ou être imprégnée sur une lingette. Ces formes d'exécution ont l'avantage de permettre un conditionnement de la composition sous-forme de mono-doses évitant l'application sur la peau d'une quantité excessive d'agent desquamant susceptible de générer des effets indésirables. Elles permettent en outre de respecter un temps de pause minimal, qui dépendra de la nature et de la concentration de l'agent desquamant, assurant l'obtention du résultat voulu.

Afin de ramener la peau à un pH proche de 6 et de l'apaiser, la seconde étape du procédé selon l'invention est suivie de l'application sur la peau d'une composition comprenant une eau thermale ou minérale ayant une minéralisation d'au moins 300 mg/l.

On entend, dans l'invention, par "minéralisation", la somme des concentrations en anions et en cations présents dans l'eau thermale ou minérale. Le fait d'utiliser une eau de forte minéralisation permet de compenser l'effet irritant des particules d'oxyde d'aluminium et des agents desquamants mis en oeuvre précédemment.

Dans la présente invention, on peut utiliser indifféremment une eau thermale ou une eau minérale. En général, une eau minérale est propre à la consommation, ce qui n'est pas toujours le cas d'une eau thermale. Chacune de ces eaux contient, entre autres, des minéraux solubilisés et des oligo-éléments.

L'eau thermale et/ou minérale utilisée selon l'invention peut avoir une minéralisation d'au moins 400 mg/l, en particulier d'au moins 700 mg/l et, plus particulièrement, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau d'Avène, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.

Parmi ces eaux, celles qui présentent une minéralisation inférieure à 700 mg/l mais supérieure 400 mg/l sont l'eau de la Roche Posay, les Eaux Bonnes, l'eau de Saint Christau.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy et l'eau d'Uriage.

Les eaux contenant au moins 9 mg/l d'oxyde de silicium sont l'eau de la Roche Posay, l'eau de Vittel, les eaux du bassin de Vichy, l'eau d'Uriage.

Les eaux du bassin de Vichy sont préférées pour une utilisation dans la présente invention.

La composition apaisante mise en oeuvre dans la troisième étape du procédé selon l'invention contient généralement plus de 40% en poids, de préférence plus de 60% en poids, plus préférentiellement plus de 80% en poids, voire 100% en poids d'une eau minérale ou thermale telle que définie ci-dessus.

Après cette étape dite de neutralisation, la quatrième étape du procédé selon l'invention est une étape dite d'entretien, comprenant l'application sur la peau d'une composition anti-âge renfermant au moins un actif anti-âge choisi parmi :
- les composés augmentant la synthèse de collagène (tels que les extraits de *Centella asiatica ;* les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®} ; et les hormones végétales telles que les auxines et les lignanes) et/ou d'élastine (tels que l'extrait de *Saccharomyces Cerivisiae* commercialisé par la société LSN sous la dénomination commerciale Cytovitin^{®} ; et l'extrait d'algue *Macrocystis pyrifera* commercialisé par la société SECMA sous la dénomination commerciale Kelpadelie^{®}) et/ou des glycosaminoglycannes (tels que le produit de fermentation du lait par *Lactobacillus vulgaris*, commercialisé par la société BROOKS sous la dénomination commerciale Biomin yogourth^{®} ; l'extrait d'algue brune *Padina pavonica* commercialisé par la société ALBAN MÜLLER sous la dénomination commerciale HSP3^{®} ; et l'extrait de *Saccharomyces cerevisiae* disponible notamment auprès de la société SILAB sous la dénomination commerciale Firmalift^{®} ou auprès de la société LSN sous la dénomination commerciale Cytovitin^{®}) et/ou des protéoglycannes et/ou de la fibronectine (tels que l'extrait de zooplancton Salina commercialisé par la société SEPORGA sous la dénomination commerciale GP4G^{®} ; l'extrait de levure disponible notamment auprès de la société ALBAN MÜLLER sous la dénomination commerciale Drieline^{®} ; et le palmitoyl pentapeptide commercialisé par la société SEDERMA sous la dénomination commerciale Matrixil^{®}) et/ou de la laminine ;
- les composés inhibant la dégradation du collagène (tels que les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja commercialisés par exemple par la société ICHIMARU PHARCOS sous la dénomination commerciale Flavostérone SB^{®}, de trèfle rouge, de lin, de kakkon ou de sauge) et/ou de l'élastine (tels que l'extrait peptidique de graines de *Pisum sativum* commercialisé par la société LSN sous la dénomination commerciale Parelastyl^{®} ; les héparinoïdes ; et les pseudodipeptides tels que l'acide {2-[acétyl-(3-trifluorométhyl-phényl)-amino]-3-méthyl-butyrylamino} acétique) ;
- les agents dermo-décontractants, tels que l'alvérine et ses sels, les sels de manganèse et en particulier le gluconate de manganèse, les sels de magnésium et en particulier le gluconate et le sulfate de magnésium, l'hexapeptide Argireline R commercialisé par la société LIPOTEC, l'adénosine, ainsi que les sapogénines et les extraits naturels, en particulier de *Dioscorea opposita* ou de *Dioscorea villosa* (Wild Yam) en contenant, ainsi que les extraits de *Boswellia serrata* ;
- les agents inhibant la glycation des protéines, tels que les extraits végétaux de la famille des Ericaceae, notamment un extrait de myrtille (*Vaccinium angusfifollium*) ; l'ergothionéine et ses dérivés ; et les hydroxystilbènes et leurs dérivés, tels que le resvératrol et le 3,3', 5,5'-tétrahydroxystilbène ;
- les agents augmentant la prolifération des kératinocytes (tels que les rétinoïdes dont le rétinol et le palmitate de rétinyle, l'adénosine, le phloroglucinol, les extraits de tourteaux de noix commercialisés par la société GATTEFOSSE et les extraits de *Solanum tuberosum* commercialisés par la société SEDERMA) et/ou des fibroblastes (tels que les protéines ou polypeptides végétaux, extraits notamment du soja, et les hormones végétales telles que les giberrellines et les cytokinines) ;
- les agents augmentant la différenciation des kératinocytes, tels que les minéraux dont le calcium ; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol ;
et leurs mélanges.

Parmi ces composés, on préfère tout particulièrement l'acide ascorbique et/ou ses dérivés tels que le glucoside d'ascorbyle, l'adénosine et leurs mélanges.

L'actif anti-âge peut représenter de 0,001 à 10%, de préférence de 0,01 à 5%, du poids total de la composition mise en oeuvre dans la quatrième étape du procédé selon l'invention.

Cette composition comprend généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner la consommatrice d'utiliser cette composition. Le pH de cette composition, qui est avantageusement proche de celui de la peau, est généralement compris entre 5 et 7 et va de préférence de 5,5 à 6,5.

Les compositions mise en oeuvres dans les différentes étapes du procédé selon l'invention peuvent se présenter sous toutes les formes galéniques classiquement utilisées pour une application topique et notamment sous forme de dispersions du type lotion ou gel, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, les compositions de microdermabrasion et/ou anti-âge (respectivement mises en oeuvre dans la première et/ou dans la quatrième étape) se présentent sous la forme d'une émulsion huile-dans-eau (H/E). Les huiles présentes dans ces émulsions peuvent être des huiles de silicone, volatiles ou non, des huiles hydrocarbonées, des huiles végétales. Ces émulsions peuvent en outre comprendre des corps gras non huileux, tels que du beurre de karité, des gommes de silicone, des esters d'acides gras et d'alcools gras, des acides gras et des alcools gras.

En outre, la composition de peeling mise en oeuvre dans la seconde étape se présente de préférence sous la forme d'un gel aqueux.

Enfin, la composition apaisante mise en oeuvre dans la troisième étape se présente de préférence sous une forme vaporisable.

Ces compositions peuvent en outre contenir divers adjuvants couramment utilisés dans le domaine cosmétique, tels que des émulsionnants dont les esters d'acide gras et de glycéryle, les esters d'acides gras et de sucre, les esters d'acide gras et de sorbitane, les esters d'acide gras et polyéthylèneglycol, les alcools gras éthoxylés et les alkylpolyglycosides ; des charges, notamment des fibres et/ou des microbilles de polyacrylamide (Nylon), de la silice, éventuellement sous forme de dispersion colloïdale, et/ou des microsphères organiques éventuellement expansées ; des conservateurs et/ou des co-conservateurs tels que le caprylyl glycol ; des séquestrants tels que les sels d'EDTA ; des colorants ; des parfums ; des ajusteurs de pH tels que des neutralisants et/ou des tampons ; de l'éthanol ; et des épaississants et gélifiants, en particulier les homo- et copolymères d'acrylamide, les homo- et copolymères acryliques, les homo- et copolymères d'acide acrylamidométhylpropane sulfonique (AMPS) et la gomme de xanthane.

Bien entendu, l'homme du métier veillera à choisir ce ou ces éventuels composés additionnels et/ou leur quantité de manière telle que les propriétés avantageuses des compositions selon l'invention ne soient pas, ou substantiellement pas, altérées par l'adjonction envisagée.

En outre, la composition mise en oeuvre dans la quatrième étape du procédé selon l'invention contient de préférence des additifs permettant de protéger la peau, précédemment fragilisée, vis-à-vis de nouvelles agressions, en particulier environnementales.

Elle contient donc avantageusement au moins un agent hydratant tel que les polyols, dont la glycérine, au moins un agent anti-oxydant ou anti-radicalaire tel que le tocophérol, l'acétate de tocophérol, l'acide ascorbique et le pyrrolidone carboxylate d'arginine, au moins un agent photoprotecteur organique ou inorganique actif dans l'UVA et/ou l'UVB, ou un mélange de tels agents.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Les agents photoprotecteurs organiques plus particulièrement préférés sont choisis parmi les composés suivants (en noms CTFA ou en noms chimiques) :
- Ethylhexyl Salicylate,
- Ethylhexyl Methoxycinnamate
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de düsobutyle)-s-triazine
- Anisotriazine,
- Ethylhexyl triazone,
- Diethylhexyl Butamido Triazone,
- Methylène bis-Benzotriazolyl Tetramethylbutylphénol
- Drometrizole Trisiloxane
- Polysilicone-15
- le 1,1-dicarboxy (2,2'-diméthyl-propyl)-4,4-diphénylbutadiène
- la 2,4-bis-[5-1(diméthylpropyl)benzoxazol-2-yl-(4-phenyl)-imino]-6-(2-ethylhexyl)-imino-1,3,5-triazine et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs peuvent être présents dans la quatrième composition selon l'invention dans des proportions allant de 0,1 à 20% en poids par rapport au poids total de la composition, et de préférence allant de 0,2 à 15% en poids par rapport au poids total de la composition.

Les compositions utilisées dans le procédé selon l'invention peuvent être disposées dans un conditionnement commun ou "kit".

La présente invention a également pour objet un kit comprenant :
- un premier conditionnement renfermant une composition qui contient au moins 5% en poids de particules d'oxyde métallique,
- au moins un deuxième conditionnement renfermant une composition de peeling qui contient au moins 5% en poids d'au moins un agent desquamant,
- un troisième conditionnement renfermant une composition contenant une eau thermale ou minérale ayant une minéralisation d'au moins 300 mg/l,
- un quatrième conditionnement renfermant une composition qui contient au moins un actif anti-âge choisi parmi : les composés augmentant la synthèse de collagène et/ou d'élastine et/ou des glycosaminoglycannes et/ou des protéoglycannes et/ou de lafibronectine et/ou de la laminine, les composés inhibant la dégradation du collagène et/ou de l'élastine, les agents dermo-décontractants, les agents inhibant la glycation des protéines, les agents augmentant la prolifération des kératinocytes et/ou des fibroblastes, les agents augmentant la différenciation des kératinocytes, et leurs mélanges.

Les termes "premier", "deuxième", "troisième" et "quatrième" conditonnements utilisés ci-dessus n'ont pas pour effet de différencier les conditionnements sur le plan de leur apparence, mais sur le plan de la composition qu'ils contiennent. Certains ou tous peuvent donc se présenter sous la même forme physique.

Toutefois, selon une forme d'exécution préférée de l'invention, le premier conditionnement peut se présenter sous forme de tube, de préférence ayant une contenance allant de 30 à 40 ml.

Le deuxième conditionnement peut être un sachet contenant une lingette sur laquelle est imprégnée la deuxième composition. Le kit selon l'invention peut par exemple contenir trois sachets de ce type.

Le troisième conditionnement peut se présenter sous forme de dispositif aérosol contenant l'eau thermale ou minérale, et un gaz propulseur qui peut être n'importe quel gaz liquéfiable utilisé habituellement dans des dispositifs aérosol tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅. On préfère tout particulièrement utiliser comme agent propulseur les alcanes en C₃₋₅, et en particulier le propane, le n-butane et l'isobutane. Ce dispositif peut ainsi avoir une contenance de 25 à 100 ml, avantageusement de 50 ml, par exemple.

Le quatrième conditionnement peut se présenter sous forme de flacon-pompe, de tube ou de pot, par exemple, qui peut notamment avoir une contenance de 30 à 50 ml.

Le kit décrit ci-dessus peut également contenir une notice renfermant des indications sur son mode d'utilisation, pour la mise en oeuvre du procédé décrit précédemment.

Un mode d'utilisation préférentiel, encore désigné par "routine", comprend :
- la mise en oeuvre du procédé décrit précédemment le matin d'un jour de semaine, et
- l'application sur la peau de la quatrième composition les six jours suivants,
ces deux étapes étant répétées pendant trois à quatre semaines consécutives.

La routine elle-même peut être mise en oeuvre de une à quatre fois par an, idéalement à chaque saison. Elle peut être utilisée pour préparer la peau à la réalisation d'un peeling dermatologique effectué par un praticien, ou pour entretenir les effets d'un tel peeling après qu'il ait été réalisé.

Chacune des deux étapes de cette routine peut être suivie de l'application d'une composition de maquillage.

L'invention sera maintenant illustrée par les exemples non limitatifs suivants. Dans ces exemples, les quantités sont indiquées en pourcentage pondéral.

### EXEMPLES

### Exemple 1 : kit anti-âge

Un kit peut être été fabriqué, qui comprend, outre un dispositif aérosol contenant 50ml d'eau de Vichy, un tube renfermant la composition A de microdermabrasion (première composition selon l'invention), la composition B de peeling (deuxième composition selon l'invention) et la composition C à effet anti-âge (quatrième composition selon l'invention) décrites ci-dessous.

### Composition A de microdermabrasion (crème)

On prépare une émulsion H/E ayant la composition ci-dessous, de façon classique pour l'homme du métier :

| | | | | |
|---|---|---|---|---|
| *Phase A* | Stéarate de PEG (100 OE) | | 0,75 | % |
| | Stéarate de glycéryle | | 0,75 | % |
| | Alcool stéarylique | | 1,5 | % |
| | Huile minérale | | 10 | % |
| | Huile d'amande d'abricots | | 2 | % |
| | Polydiméthylsiloxane | | 1 | % |
| | | | | |
| *Phase B* | Eau déminéralisée | qsp. | 100 | % |
| | Polyholoside à 1%* | | 1 | % |
| | Polyacrylamide | | 0,3 | % |
| | Butylène glycol | | 3 | % |
| | Propylène glycol | | 3 | % |
| | | | | |
| *Phase C* | Oxyde d'aluminium** | | 15 | % |
| | | | | |
| *Phase D* | Eau déminéralisée | | 5 | % |
| | Conservateurs | | qs | |

| | | | | |
|---|---|---|---|---|
| * Fucogel 1000 PP de SOLABIA ** Dermagrain 900 de MarkeTech International | | | | |

Cette crème peut être appliquée le matin sur le visage mouillé, en évitant le contour des yeux et des lèvres, par massage du bout des doigts pendant environ 1 minute 30, suivi d'un rinçage à l'eau et d'un séchage à l'aide d'une serviette propre. Elle permet d'améliorer l'éclat du teint, de lisser sa surface et de réduire l'apparence des rides. Elle prépare la peau à l'application de la composition de peeling.

### Composition B de peeling (gel aqueux)

On imprègne une lingette à l'aide de la composition suivante :

| | | | |
|---|---|---|---|
| Acide glycolique | | 9,5 | % |
| Acide lactique | | 0,45 | % |
| Gélifiant | | 0,25 | % |
| Ethanol | | 5 | % |
| Neutralisant | | 3,4 | % |
| Eau | qsp | 100 | % |

La lingette est laissée environ 3 minutes en contact avec la peau. Cette composition permet de resserrer les pores, corriger les rides et ridules et accélérer le renouvellement cutané. Cette étape est suivie de l'application sur la peau d'une eau minérale, telle que l'eau du bassin de VICHY, par exemple sous forme de brumisateur, puis du séchage de la peau à l'aide d'une serviette propre, en vue d'apaiser la peau

### Composition C à effet anti-âge (émulsion H/E)

| | | | | |
|---|---|---|---|---|
| Tocophérol | | | 0,025 | % |
| Arginine PCA | | | 0,1 | % |
| Glycérine | | | 7 | % |
| Tensioactifs | | | 2,5 | % |
| Huiles | | | 8 | % |
| Filtres UV | | | 10 | % |
| Epaississants | | | 1 | % |
| Alcool cétylique | | | 2 | % |
| Acides gras | | | 1 | % |
| Myristate de myristyle | | | 2 | % |
| EDTA disodique | | | 0,1 | % |
| Conservateurs | | | 1 | % |
| Ethanol | | | 4 | % |
| Neutralisant | | qs | pH 6 | |
| Eau | qsp | | 100 | % |

Cette composition permet de lisser les rides, raffermir la peau et lui redonner de l'éclat.

### Exemple 2 : Composition A' de microdermabrasion (gel moussant)

On prépare la composition suivante de façon classique pour l'homme du métier :

| | | | | |
|---|---|---|---|---|
| *Phase A* | Eau déminéralisée | qsp | 100 | % |
| | EDTA | | 0,2 | % |
| | Conservateur | | qs | |
| | Copolymère acrylique (PEMULEN TR1) | | 1 | % |
| | Sodium lauroyl sarcosinate | | 2 | % |
| | Sodium laureth sulfate | | 5 | % |
| | | | | |
| *Phase B* | Eau déminéralisée | | 10 | % |
| | Polyholoside à 1 %* | | 2 | % |
| | Gomme-de-xanthane | | 0,3 | % |
| | Butylène glycol | | 3 | % |
| | Propylène glycol | | 3 | % |
| | Conservateur | | qs | |
| | Triéthanolamine | | 0,8 | % |
| | Oxyde de magnésium** | | 6 | % |

| | | | | |
|---|---|---|---|---|
| * Fucogel 1000 PP de SOLABIA ** Magnaderm 100 de MARKETECH INTERNATIONAL | | | | |

## Revendications

1. Procédé cosmétique de soin de la peau, destiné à atténuer les signes cutanés du vieillissement, comprenant l'application successive sur une zone de peau préalablement mouillée :
- d'une composition de microdermabrasion renfermant au moins 5% en poids de particules d'oxyde métallique, qui est appliquée par massage, puis éliminée par lavage,
- d'une composition de peeling renfermant au moins 3% en poids d'au moins un agent desquamant,
- d'une composition apaisante comprenant une eau thermale ou minérale ayant une minéralisation d'au moins 300 mg/l ,
- d'une composition anti-âge renfermant au moins un actif anti-âge choisi parmi : les composés augmentant la synthèse de collagène et/ou d'élastine et/ou des glycosaminoglycannes et/ou des protéoglycannes et/ou de la fibronectine et/ou de la laminine ; les composés inhibant la dégradation du collagène et/ou de l'élastine ; les agents dermo-décontractants ; les agents inhibant la glycation des protéines ; les agents augmentant la prolifération des kératinocytes et/ou des fibroblastes ; les agents augmentant la différenciation des kératinocytes ; et leurs mélanges.

2. Procédé selon la revendication 1, **caractérisé en ce que** ladite zone de peau est une zone de peau du visage.

3. Procédé selon la revendication 1 ou 2, **caractérisé en ce que** les particules d'oxyde métallique sont des particules d'oxyde de magnésium ou d'aluminium.

4. Procédé selon l'une quelconque des revendications 1 à 3, **caractérisé en ce que** les particules d'oxyde métallique ont une taille moyenne de particule allant de 100 à 180 µm.

5. Procédé selon l'une quelconque des revendications 1 à 4, **caractérisé en ce que** l'oxyde métallique représente de 10 à 30% du poids total de la composition de microdermabrasion.

6. Procédé selon l'une quelconque des revendications 1 à 5, **caractérisé en ce que** la composition de microdermabrasion a un pH compris entre 5,5 et 7.

7. Procédé selon l'une quelconque des revendications 1 à 6, **caractérisé en ce que** la composition de microdermabrasion renferme au moins un polyholoside hétérogène.

8. Procédé selon l'une quelconque des revendications 1 à 7, **caractérisé en ce que** l'agent desquamant est choisi parmi-: les α-hydroxyacides tels que l'acide citrique, lactique, glycolique, malique, tartrique ou mandélique ; les β-hydroxyacides tels que l'acide salicylique ou l'acide n-octanoyl-5-salicylique ; l'urée ; les composés aminosulfoniques et en particulier l'acide (N-2 hydroxyéthylpiperazine-N-2-éthane) sulfonique (HEPES) ; et les dérivés de l'acide 2-oxothiazolidine-4-carboxylique (procystéine).

9. Procédé selon l'une quelconque des revendications 1 à 8, **caractérisé en ce que** la composition de peeling a un pH compris entre 3,5 et 4,5.

10. Procédé selon l'une quelconque des revendications 1 à 9, **caractérisé en ce que** la composition de peeling est imprégnée sur une lingette.

11. Procédé selon l'une quelconque des revendications 1 à 10, **caractérisé en ce que** la composition apaisante contient 100% en poids d'eau minérale ou thermale.

12. Procédé selon l'une quelconque des revendications 1 à 11, **caractérisé en ce que** l'actif anti-âge est choisi parmi : l'acide ascorbique et/ou ses dérivés tels que le glucoside d'ascorbyle, l'adénosine et leurs mélanges.

13. Procédé selon l'une quelconque des revendications 1 à 12, **caractérisé en ce que** la composition anti-âge a un pH compris entre 5,5 et 6,5.

14. Procédé selon l'une quelconque des revendications 1 à 13, **caractérisé en ce que** la composition de microdermabrasion et/ou la composition anti-âge sont sous la formule d'une émulsion huile-dans-eau.

15. Procédé selon l'une quelconque des revendications 1 à 14, **caractérisé en ce que** la composition de peeling se présente sous la forme d'un gel aqueux.

16. Procédé selon l'une quelconque des revendications 1 à 15, **caractérisé en ce que** la composition anti-âge contient au moins un agent hydratant tel que les polyols, dont la glycérine, au moins un agent anti-oxydant ou anti-radicalaire tel que le tocophérol, l'acétate de tocophérol, l'acide ascorbique et le pyrrolidone carboxylate d'arginine, au moins un agent photoprotecteur organique ou inorganique actif dans l'UVA et/ou l'UVB, ou un mélange de tels agents.

17. Kit comprenant :
- un premier conditionnement renfermant une composition qui contient au moins 5% en poids de particules d'oxyde métallique,
- au moins un deuxième conditionnement renfermant une composition de peeling qui contient au moins 5% en poids d'au moins un agent desquamant,
- un troisième conditionnement renfermant une composition contenant une eau thermale ou minérale ayant une minéralisation d'au moins 300 mg/l,
- un quatrième conditionnement renfermant une composition qui contient au moins un actif anti-âge choisi parmi : les composés augmentant la synthèse de collagène et/ou d'élastine et/ou des glycosaminoglycannes et/ou des protéoglycannes et/ou de lafibronectine et/ou de la laminine, les composés inhibant la dégradation du collagène et/ou de l'élastine, les agents dermo-décontractants, les agents inhibant la glycation des protéines, les agents augmentant la prolifération des kératinocytes et/ou des fibroblastes, les agents augmentant la différenciation des kératinocytes, et leurs mélanges.

## Claims

1. Cosmetic method for caring for the skin, intended to soften cutaneous signs of ageing, comprising the successive application, to an area of skin wetted beforehand:
- of a microdermabrasion composition including at least 5% by weight of metal oxide particles, which is applied by massaging, and then removed by washing,
- of a peeling composition including at least 3% by weight of at least one desquamating agent,
- of a soothing composition comprising a thermal or mineral water having a mineral content of at least 300 mg/l,
- of an anti-ageing composition including at least one anti-ageing active principle chosen from: compounds which enhance the synthesis of collagen and/or of elastin and/or of glycosaminoglycans and/or of proteoglycans and/or of fibronectin and/or laminin; compounds which inhibit the decomposition of collagen and/or of elastin; skin relaxants; agents which inhibit the glycation of proteins; agents which enhance the proliferation of keratinocytes and/or of fibroblasts; agents which enhance the differentiation of keratinocytes; and their mixtures.

2. Method according to Claim 1, **characterized in that** the said area of skin is an area of skin of the face.

3. Method according to Claim 1 or 2, **characterized in that** metal oxide particles are magnesium oxide particles or aluminium oxide particles.

4. Method according to any one of Claims 1 to 3, **characterized in that** metal oxide particles have a mean particle size ranging from 100 to 180 µm.

5. Method according to any one of Claims 1 to 4, **characterized in that** the metal oxide represents from 10 to 30% of the total weight of the microdermabrasion composition.

6. Method according to any one of Claims 1 to 5, **characterized in that** the microdermabrasion composition has a pH of between 5.5 and 7.

7. Method according to any one of Claims 1 to 6, **characterized in that** the microdermabrasion composition includes at least one heterogeneous polysaccharide.

8. Method according to any one of Claims 1 to 7, **characterized in that** the desquamating agent is chosen from α-hydroxy acids, such as citric, lactic, glycolic, malic, tartaric or mandelic acid; β-hydroxy acids, such as salicylic acid or 5-(n-octanoyl)-salicylic acid; urea; aminosulphonic compounds and in particular N-(2-hydroxyethyl)piperazine-N'-2-ethanesulphonic acid (HEPES); and derivatives of 2-oxothiazolidine-4-carboxylic acid (procysteine).

9. Method according to any one of Claims 1 to 8, **characterized in that** the peeling composition has a pH of between 3.5 and 4.5.

10. Method according to any one of Claims 1 to 9, **characterized in that** the peeling composition is impregnated on a wipe.

11. Method according to any one of Claims 1 to 10, **characterized in that** the soothing composition comprises 100% by weight of mineral or thermal water.

12. Method according to any one of Claims 1 to 11, **characterized in that** the anti-ageing active principle is chosen from: ascorbic acid and/or its derivatives, such as ascorbyl glucoside, adenosine and their mixtures.

13. Method according to any one of Claims 1 to 12, **characterized in that** the anti-ageing composition has a pH of between 5.5 and 6.5.

14. Method according to any one of Claims 1 to 13, **characterized in that** the microdermabrasion composition and/or the anti-ageing composition are in the form of an oil-in-water emulsion.

15. Method according to any one of Claims 1 to 14, **characterized in that** the peeling composition is provided in the form of an aqueous gel.

16. Method according to any one of Claims 1 to 15, **characterized in that** the anti-ageing composition comprises at least one moisturizing agent, such as polyols, including glycerol, at least one antioxidant or agent for combating free radicals, such as tocopherol, tocopherol acetate, ascorbic acid and arginine pyrrolidonecarboxylate, at least one organic or inorganic photoprotective agent which is active in the UV-A and/or UV-B region, or a mixture of such agents.

17. Kit comprising:
- a first packaging including a composition which comprises at least 5% by weight of metal oxide particles,
- at least one second packaging including a peeling composition which comprises at least 5% by weight of at least one desquamating agent,
- a third packaging including a composition comprising a thermal or mineral water having a mineral content of at least 300 mg/l,
- a fourth packaging including a composition which comprises at least one anti-ageing active principle chosen from: compounds which enhance the synthesis of collagen and/or of elastin and/or of glycosaminoglycans and/or of proteoglycans and/or of fibronectin and/or of laminin, compounds which inhibit the decomposition of collagen and/or of elastin, skin relaxants, agents which inhibit the glycation of proteins, agents which enhance the proliferation of keratinocytes and/or of fibroblasts, agents which enhance the differentiation of keratinocytes, and their mixtures.

## Patentansprüche

1. Kosmetisches Verfahren zur Pflege der Haut, das für die Abschwächung der Zeichen der Hautalterung vorgesehen ist, das die aufeinanderfolgende Anwendung
- einer Zusammensetzung für die Mikrodermabrasion, die mindestens 5 Gew.-% Metalloxidpartikel enthält, die durch Massage angewendet und anschließend durch Waschen entfernt wird,
- einer Peeling-Zusammensetzung, die mindestens 3 Gew.-% mindestens eines abschuppenden Mittels enthält,
- einer beruhigenden Zusammensetzung, die ein Thermalwasser oder Mineralwasser enthält, das einen Mineralstoffgehalt von mindestens 300 mg/l hat,
- einer Zusammensetzung gegen die Alterung, die mindestens einen Anti-Age-Wirkstoff enthält, der ausgewählt wird unter: den Verbindungen, die die Synthese von Collagen und/oder Elastin und/oder Glykosaminoglykanen und/oder Proteoglykanen und/oder Fibronectin und/oder Laminin verstärken; den Verbindungen, die den Abbau von Collagen und/oder Elastin hemmen; den hautstraffenden Mittel,n; den Mitteln, die die Glykation von Proteinen hemmen; den Mitteln, die die Proliferation von Keratinocyten und/ oder Fibroblasten verstärken; den Mitteln, die die Differenzierung von Keratinocyten verstärken; und deren Gemischen,
auf einen zuvor befeuchteten Hautbereich umfasst.

2. Verfahren nach Anspruch 1, **dadurch gekennzeichnet, dass** es sich bei dem Hautbereich um einen Bereich der Gesichtshaut handelt.

3. Verfahren nach Anspruch 1 oder 2, **dadurch gekennzeichnet, dass** die Metalloxidpartikel Magnesiumoxidpartikel oder Aluminiumoxidpartikel sind.

4. Verfahren nach einem der Ansprüche 1 bis 3, **dadurch gekennzeichnet, dass** die Metalloxidpartikel eine mittlere Partikelgröße haben, die im Bereich von 100 bis 180 µm liegt.

5. Verfahren nach einem der Ansprüche 1 bis 4, **dadurch gekennzeichnet, dass** das Metalloxid 10 bis 30 % des Gesamtgewichts der Zusammensetzung für die Mikrodermabrasion ausmacht.

6. Verfahren nach einem der Ansprüche 1 bis 5, **dadurch gekennzeichnet, dass** die Zusammensetzung für die Mikrodermabrasion eine pH-Wert hat, der im Bereich von 5,5 bis 7 liegt.

7. Verfahren nach einem der Ansprüche 1 bis 6, **dadurch gekennzeichnet, dass** die Zusammensetzung für die Mikrodermabrasion mindestens ein heterogenes Polyholosid enthält.

8. Verfahren nach einem der Ansprüche 1 bis 7, **dadurch gekennzeichnet, dass** das abschuppende Mittel ausgewählt wird unter: den α-Hydroxysäuren, wie Citronensäure, Milchsäure, Glycolsäure, Äpfelsäure, Weinsäure oder Mandelsäure; den β-Hydroxysäuren, wie Salicylsäure oder 5-n-Octanoylsalicylsäure; Harnstoff; den Aminosulfonverbindungen und vor allem der (N-2-Hydroxyethylpiperazin-N-2-ethan)-sulfonsäure (HEPES); und den Derivaten der 2-Oxothiazolidin-4-carbonsäure (Procystcin).

9. Verfahren nach einem der Ansprüche 1 bis 8, **dadurch gekennzeichnet, dass** die Peehng-Zusammcnsetzung eine pH-Wert hat, der im Bereich von 3,5 bis 4,5 liegt.

10. Verfahren nach einem der Ansprüche 1 bis 9, **dadurch gekennzeichnet, dass** ein Tuch mit der Peeling-Zusamznensetzung imprägniert wird.

11. Verfahren nach einem der Ansprüche 1 bis 10, **dadurch gekennzeichnet, dass** die beruhigende Zusammensetzung 100 Gew.-% Mineralwasser oder Thermalwasser enthält.

12. Verfahren nach einem der Ansprüche 1 bis 11, **dadurch gekennzeichnet, dass** der Anti-Age-Wirkstoff ausgewählt wird unter: Ascorbinsäure und/oder ihren Derivaten, wie Ascorbylglucosid, Adenosin, und deren Gemischen.

13. Verfahren nach einem der Ansprüche 1 bis 12, **dadurch gekennzeichnet, dass** die Zusammensetzung gegen die Alterung einen pH-Wert hat, der im Bereich von 5,5 bis 6,5 liegt.

14. Verfahren nach einem der Ansprüche 1 bis 13, **dadurch gekennzeichnet, dass** die Zusammensetzung für die Mikrodermabrasion und/oder die Zusammensetzung gegen die Alterung in Form einer Öl-in-Wasser-Emulsion vorliegen.

15. Verfahren nach einem der Ansprüche 1 bis 14, **dadurch gekennzeichnet, dass** die Peeling-Zusammensetzung in Form eines wässrigen Gels vorliegt.

16. Verfahren nach einem der Ansprüche 1 bis 15, **dadurch gekennzeichnet, dass** die Zusammensetzung gegen die Alterung mindestens ein Hydratisicrungsmittel wie Polyole, darunter Glycerin, mindestens ein Antioxidationsmittel oder Mittel gegen Radikale, wie Tocopherol, Tocopherolacetat, Ascorbinsäure und Argininpyrrolidoncarboxylat, mindestens ein organisches oder anorganisches Lichtschutzmittel, das im UVA und/ oder UVB wirksam ist, oder ein Gemisch derartiger Mittel enthält.

17. Kit, der umfasst:
- eine erste Verpackung, die eine Zusammensetzung enthält, die mindestens 5 Gew.-% Metalloxidpartikel enthält,
- mindestens eine zweite Verpackung, die eine Peeling-Zusammensetzung enthält, die mindestens 5 Gew.-% mindestens eines abschuppenden Mittels enthält,
- eine dritte Verpackung, die eine Zusammensetzung enthält, die ein Thermalwasser oder Mineralwasser enthält, das einen Mineralstoffgehalt von mindestens 300 mg/l hat,
- eine vierte Verpackung, die eine Zusammensetzung enthält, die mindestens einen Anti-Age-Wirkstoff enthält, der ausgewählt wird unter: den Verbindungen, die die Synthese von Collagen und/oder Elastin und/oder Glykosaminoglykanen und/oder Proteoglykanen und/oder von Fibronektin und/oder Laminin verstärken; den Verbindungen, die den Abbau von Collagen und/oder Elastin hemmen; den hautstraffenden Mitteln; den Mitteln, die die Glykation von Proteinen hemmen; den Mitteln, die die Proliferation von Keratinocyten und/ oder Fibroblasten verstärken; den Mitteln, die die Differenzierung von Keratinocyten verstärken; und deren Gemischen.
